(19) Europäisches Patentamt
European Patent Office
Office européen des brevets

(11) Veröffentlichungsnummer: **0 157 184**
**B1**

(12) # EUROPÄISCHE PATENTSCHRIFT

(45) Veröffentlichungstag der Patentschrift: **07.01.88**

(21) Anmeldenummer: **85102327.5**

(22) Anmeldetag: **01.03.85**

(51) Int. Cl.[4]: **C 07 D 335/04**, C 07 D 319/08, C 07 D 339/08, C 07 D 221/20, C 07 C 131/08, A 01 N 33/24, A 01 N 43/16, A 01 N 43/18, A 01 N 43/32, A 01 N 43/42

(54) **Cyclohexenone, Verfahren zu ihrer Herstellung sowie ihre Verwendung zur Bekämpfung unerwünschten Pflanzenwuchses.**

(30) Priorität: **06.03.84 DE 3408153**
**22.03.84 DE 3410492**

(43) Veröffentlichungstag der Anmeldung:
**09.10.85 Patentblatt 85/41**

(45) Bekanntmachung des Hinweises auf die Patenterteilung:
**07.01.88 Patentblatt 88/1**

(84) Benannte Vertragsstaaten:
**BE DE FR GB**

(56) Entgegenhaltungen:
**AT - B - 335 419**
**DE - A - 2 318 676**
**DE - A - 3 146 309**
**DE - A - 3 239 071**

**PATENT ABSTRACTS OF JAPAN, unexamined applications, Field C, Band 7, Nr. 28, 4. Februar 1983, THE PATENT OFFICE JAPANESE GOVERNMENT, Seite 92 C 149**

(73) Patentinhaber: **BASF Aktiengesellschaft, Carl-Bosch-Strasse 38, D-6700 Ludwigshafen (DE)**

(72) Erfinder: **Becker, Rainer, Dr., Im Haseneck 22, D-6702 Bad Duerkheim (DE)**
Erfinder: **Jahn, Dieter, Dr., Burgunderweg 8, D-6803 Edingen-Neckarhausen (DE)**
Erfinder: **Schirmer, Ulrich, Dr., Berghalde 79, D-6900 Heidelberg (DE)**
Erfinder: **Keil, Michael, Dr., Fontanestrasse 4, D-6713 Freinsheim (DE)**
Erfinder: **Wuerzer, Bruno, Dr. Dipl.-Landwirt, Ruedigerstrasse 13, D-6701 Otterstadt (DE)**
Erfinder: **Meyer, Norbert, Dr., Dossenheimer Weg 22, D-6802 Ladenburg (DE)**

(56) Entgegenhaltungen: (Fortsetzung)
**PATENT ABSTRACTS OF JAPAN, unexamined applications, Field C, Band 7, Nr. 263, 24. November 1983, THE PATENT OFFICE JAPANESE GOVERNMENT, Seite 16 C 196**

**Die Akte enthält technische Angaben, die nach dem Eingang der Anmeldung eingereicht wurden und die nicht in dieser Patentschrift enthalten sind.**

EP 0 157 184 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Cyclohexenone, Verfahren zu ihrer Herstellung sowie Herbizide, die diese Verbindungen als Wirkstoffe enthalten.

Aus den japanischen Patentanmeldungen JP-OS 82/183 746 sowie JP-OS 83/144 384 ist bekannt, dass spirocyclische Cyclohexenone herbizid wirksam sind.

Es wurde gefunden, dass Cyclohexenone der Formel

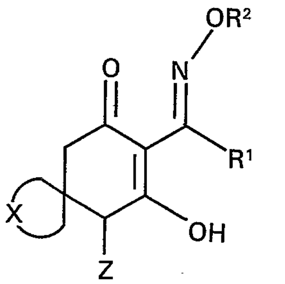

(I)

in der

$R^1$ $C_1$-$C_4$-Alkyl,

$R^2$ $C_1$-$C_4$-Alkyl, $C_3$-$C_5$-Alkinyl oder gegebenenfalls halogensubstituiertes $C_3$-$C_5$-Alkenyl,

Z Wasserstoff oder $C_2$-$C_5$-Alkoxycarbonyl und

X eine gegebenenfalls durch $C_1$-$C_4$-Alkyl, $C_1$-$C_4$-Alkoxy oder $C_1$-$C_4$-Alkylthio substituierte Pentamethylenkette, in der eine oder zwei Methylengruppen durch O, S, SO, $SO_2$ oder $NR^3$, wobei $R^3$ für $C_1$-$C_4$-Alkyl, $C_2$-$C_7$-Acyl oder Benzyl steht, ersetzt sein können, mit der Massgabe, dass ein Substituent aus der Gruppe bestehend aus Alkyl, Alkoxy und Alkylthio vorhanden ist, wenn X Pentamethylen ist, bedeuten,

sowie die Kulturpflanzen-verträglichen Salze dieser Verbindungen, eine gute herbizide Wirkung, vorzugsweise gegen Arten aus der Familie der Gräser (Gramineen), besitzen. Sie sind verträglich und somit selektiv in breitblättrigen Kulturen sowie in monokotylen Gewächsen, die nicht zu den Gramineen zählen.

Die Cyclohexenone der Formel I können in tautomeren Formen auftreten, die alle vom Patentanspruch umfasst werden:

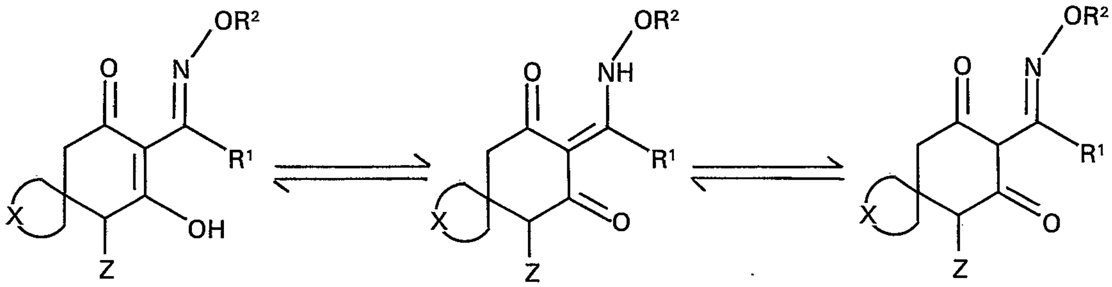

In Formel I haben die Substituenten folgende Bedeutungen

$R^1$ ist unverzweigtes oder verzweigtes $C_1$-$C_4$-Alkyl, beispielsweise Methyl, Ethyl, n-Propyl, s-Butyl, i-Butyl,

$R^2$ ist unverzweigtes oder verzweigtes $C_1$-$C_4$-Alkyl, $C_3$-$C_5$-Alkinyl oder gegebenenfalls durch Halogen, insbesondere Chlor, substituiertes $C_3$-$C_5$-Alkenyl, beispielsweise Ethyl, Propyl, Allyl, Propargyl, 3-Chlorallyl (cis oder trans), 2-Chlorallyl,

Z ist Wasserstoff der $C_2$-$C_5$-Alkoxycarbonyl, beispielsweise Methoxycarbonyl, Ethoxycarbonyl,

X ist eine Pentamethylenkette, in der eine oder zwei Methylengruppen durch O, S, SO, $SO_2$ oder $NR^3$ ersetzt sein können. Dieser Rest kann gegebenenfalls durch mindestens einen Rest aus der Gruppe bestehend aus $C_1$-$C_4$-Alykl, $C_1$-$C_4$-Alkoxy und $C_1$-$C_4$-Alkylthio, beispielsweise Methyl, Ethyl, n-Propyl, s-Butyl, i-Butyl, Methoxy, Ethoxy, n-Propoxy, n-Butoxy, Methylthio, Ethylthio, i-Propylthio, s-Butylthio substituiert sein. Wenn X Pentamethylen ist, muss ein Substituent aus der Gruppe bestehend aus Alkyl, Alkoxy oder Alkylthio vorhanden sein. $R^3$ in $NR^3$ steht für $C_1$-$C_4$-Alkyl, $C_2$-$C_7$-Acyl oder Benzyl, beispielsweise für Methyl, Ethyl, Acetyl, Propinyl, Benzoyl oder Benzyl. Beispiele für X sind

$-CH_2CH(SCH_3)CH_2CH_2CH_2-$,
$-CH_2CH(SC_2H_5)CH_2CH_2CH_2-$,
$-CH_2CH(COCH_3)CH_2CH_2CH_2-$,
$-CH_2CH(OC_2H_5)CH_2CH_2CH_2-$,
$-CH_2CH_2CH(CH_3)CH_2CH_2-$,
$-CH_2CH_2OCH_2CH_2-$, $-CH_2CH_2SCH_2CH_2-$,
$-CH_2CH_2SOCH_2CH_2-$, $-CH_2OCH_2OCH_2-$,
$-CH_2CH_2SO_2CH_2CH_2-$, $-CH_2SCH_2CH_2CH_2-$,
$-CH_2SOCH_2CH_2CH_2-$, $-CH_2SO_2CH_2CH_2CH_2-$,
$-CH_2SCH_2SCH_2-$, $-CH_2SCH(CH_3)SCH_2-$,
$-CH_2SC(CH_3)_2SCH_2-$, $-CH_2CH_2NCH_3CH_2CH_2-$,
$-CH_2CH_2N(COCH_3)CH_2CH_2-$,
$-CH_2CH_2N(CH_2C_6H_5)CH_2CH_2-$,
$-CH_2CH_2N(COC_6H_5)CH_2CH_2-$.

Als Salze der Cyclohexenone der Formel I kommen beispielsweise die Alkalimetallsalze, insbesondere die Kalium- oder Natriumsalze, Erdalkalimetallsalze, insbesondere Calcium-, Magnesium- oder Bariumsalze, Mangan-, Kupfer-, Zink- oder Eisensalze sowie Ammonium-. Phosphonium-, Sulfonium und Sulfoxoniumsalze, beispielsweise Ammoniumsalze, Tetraalkylammoniumsalze, Benzyltrialkylammoniumsalze, Trialkylsulfoniumsalze, Trialkylsulfoxoniumsalze, in Betracht.

Bevorzugte Cyclohexenone der Formel I sind solche, bei denen Z Wasserstoff bedeutet, oder solche, bei denen X eine Oxapentamethylen- oder Thiapentamethylenkette bedeutet.

Die Verbindungen der Formel I können durch Umsetzung von Verbindungen der Formel

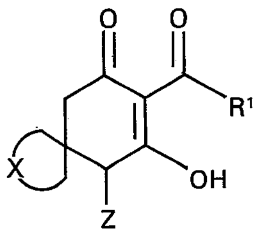

(II)

in der

$R^1$, Z und X die obengenannten Bedeutungen haben, mit Hydroxylaminderivaten $R^2O-NH_3Y$, in der $R^2$ die obengenannten Bedeutungen hat und Y ein Anion bedeutet, erhalten werden.

Man führt die Reaktion zweckmässigerweise in heterogener Phase in einem inerten Verdünnungsmittel bei einer Temperatur zwischen 0 und 80 °C oder zwischen 0 °C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Ausserdem können auch organische Basen, wie Pyridin oder tertiäre Amine, Verwendung finden.

Die Umsetzung verläuft besonders gut in einem pH-Bereich von 2 bis 9, insbesondere von 4,5 bis 5,5. Die Einstellung des pH-Bereiches erfolgt zweckmässigerweise durch Zusatz von Acetaten, beispielsweise Alkalimetallacetaten, insbesondere von Natrium- oder Kaliumacetat oder einer Mischung aus beiden Salzen. Alkalimetallacetate werden beispielsweise in Mengen von 0,5 bis 2 Mol, bezogen auf die Ammoniumverbindung der Formel $R^2O-NH_3Y$, zugesetzt.

Als Lösungsmittel sind beispielsweise Dimethylsulfoxid, Alkohole, wie Methanol, Ethanol, Isopropanol, Benzol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid und Abdestillieren des Lösungsmittels unter vermindertem Druck isoliert werden.

Die Verbindungen der Formel I können ausserdem durch Umsetzen der Verbindungen der Formel II mit Hydroxylaminen der Formel $R^2O-NH_2$, in der $R^2$ die obengenannten Bedeutungen hat, in inerten Verdünnungsmitteln bei Temperaturen zwischen 0 °C und 80 °C, insbesondere zwischen 15 und 70 °C, erhalten werden. Gegebenenfalls kann das Hydroxylamin als wässrige Lösung eingesetzt werden.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Die Verbindungen der Formel I können weiterhin durch Umsetzen von Verbindungen der Formel II mit unsubstituiertem Hydroxylammoniumsalz $NH_2OH \cdot HY$, wobei Y die obige Bedeutung hat, unter Zugabe von Lösungsmittel und Hilfsbase bei einer Temperatur zwischen 0 und 80 °C zu einem Oxim der Formel

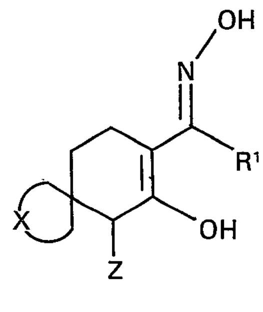

(III)

in der

$R^1$, X und Z die obengenannten Bedeutungen haben, und weitere Umsetzung mit einem Alkylierungsmittel der Formel $R^2Y'$, wobei $R^2$ die obigen Bedeutungen hat und Y' eine Abgangsgruppe bedeutet, erhalten werden.

Geeignete Lösungsmittel sind die für die Umsetzung der Verbindungen der Formel II mit Hydroxylaminen aufgeführten Lösungsmittel, geeignete Hilfsbasen sind die für die Umsetzung der Verbindungen der Formel II mit Hydroxylaminderivaten der Formel $R^2O-NH_3Y$ genannten basischen Substanzen, wobei allerdings die doppelte Menge an Base erforderlich ist.

Die Alkalimetallsalze der Verbindungen der Formel I können durch Behandeln dieser Verbindungen mit Natrium- oder Kaliumhydroxid in wässriger Lösung oder in einem organischen Lösungsmittel, wie Methanol, Ethanol, Aceton, erhalten werden. Auch Natrium- und Kaliumalkoholate können als Basen dienen.

Die übrigen Metallsalze, z.B. die Mangan-, Kupfer-, Zink-, Eisen-, Calcium-, Magnesium- und Bariumsalze können aus den Natriumsalzen durch Reaktion mit den entsprechenden Metallchloriden in wässriger Lösung hergestellt werden. Ammonium- und Phosphoniumsalze können durch Umsetzung von Verbindungen der Formel I mit Ammonium-, Phosphonium-, Sulfonium- oder Sulfoxoniumhydroxiden gegebenenfalls in wässriger Lösung erhalten werden.

Die Verbindungen der Formel II können aus Cyclohexan-1,3-dionen der Formel IV, die auch in tautomerer Form IVa vorliegen,

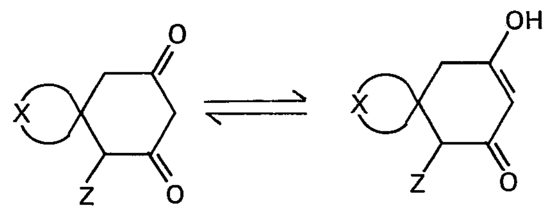

(IV) (IVa)

nach literaturbekannten Methoden [Tetrahedron Letters, 29, 2491 (1975)] hergestellt werden.

Es ist auch möglich, Verbindungen der Formel II über die Zwischenstufe der Enolester, die bei der Umsetzung von Verbindungen der Formel IV anfallen und in Gegenwart von Imidazol- oder Pyridinderivaten umgelagert werden (JP-OS 79/063052), herzustellen.

Zu den Verbindungen der Formel IV gelangt man nach literaturbekannten Verfahren, wie dies aus folgendem Schema hervorgeht

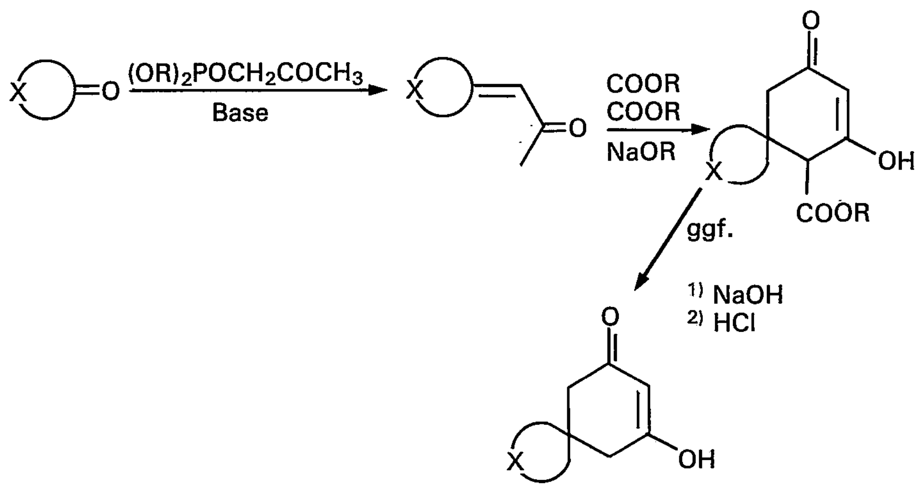


R = $C_1$-$C_4$-Alkyl

Das folgende Beispiel erläutert die Herstellung der Cyclohexenone der Formel I.

*Beispiel*

9,7 Gewichtsteile 9-Butyryl-3-oxa-spiro[5.5]undecan-8,10-dion werden in 100 Volumenteilen Ethanol aufgenommen und mit 3,57 Gewichtsteilen Natriumhydrogencarbonat versetzt. Nach Zugabe von 4,13 Gewichtsteilen Ethoxyaminohydrochlorid wird etwa 20 Stunden bei 20°C gerührt, dann wird das Reaktionsgemisch in Eiswasser/Methylenchlorid gegeben, die organische Phase wird abgetrennt und eingeengt. Es verbleiben 10,9 Gewichtsteile 9-(1-Ethoxyimino-n-butyl)-3-oxa-spiro[5.5]undecan-8,10-dion vom Schmelzpunkt 74 bis 76°C (Verbindung Nr. 1).

Die folgenden Cyclohexenonderivate der Formel I lassen sich auf gleichem Wege herstellen:

| Verbindung Nr. | X | Z | $R^1$ | $R^2$ | Fp [°C]/$n_D$ (°C)/ $^1$H-NMR-Daten |
|---|---|---|---|---|---|
| 1 | $CH_2CH_2OCH_2CH_2$ | H | n-$C_3H_7$ | $C_2H_5$ | 74-76 |
| 2 | $CH_2CH_2OCH_2CH_2$ | H | n-$C_3H_7$ | Allyl | 1,5317 (22) |
| 3 | $CH_2CH_2OCH_2CH_2$ | $COOCH_3$ | n-$C_3H_7$ | $C_2H_5$ | |
| 4 | $CO_2OCH_2CH_2CH_2$ | H | n-$C_3H_7$ | $C_2H_5$ | |
| 5 | $CH_2OCH_2CH_2CH_2$ | H | n-$C_3H_7$ | Allyl | |
| 6 | $CH_2SCH_2CH_2CH_2$ | H | n-$C_3H_7$ | $C_2H_5$ | 1,5522 (22) |
| 7 | $CH_2SCH_2CH_2CH_2$ | $COOC_2H_5$ | n-$C_3H_7$ | $C_2H_5$ | |
| 8 | $CH_2SCH_2CH_2CH_2$ | H | n-$C_3H_7$ | Allyl | 1,5572 |
| 9 | $CH_2SCH_2CH_2CH_2$ | H | n-$C_3H_7$ | 3-Chlor-allyl (trans) | 1,5657 |
| 10 | $CH_2SOCH_2CH_2CH_2$ | H | n-$C_3H_7$ | $C_2H_5$ | |
| 11 | $CH_2SO_2CH_2CH_2CH_2$ | H | n-$C_3H_7$ | $C_2H_5$ | |
| 12 | $CH_2SO_2CH_2CH_2CH_2$ | H | n-$C_3H_7$ | Allyl | |
| 13 | $CH_2CH(SEt)CH_2CH_2CH_2$ | H | n-$C_3H_7$ | $C_2H_5$ | 0,95 (t,3H); 1,2 (t,3H); 1,32 (t,3H); 2,53 (q,2H); 2,9 (t,2H); 4,1 (q,2H); 15 (s,1H) |
| 14 | $CH_2CH(SEt)CH_2CH_2CH_2$ | H | n-$C_3H_7$ | Allyl | 0,95 (t,3H); 1,2 (t,6H); 2,9 (t,2H); 4,5 (d,2H); 5,35 (dd,2H); 5,9-6,1 (m,1H) |
| 15 | $CH_2CH(OCH_3)CH_2CH_2CH_2$ | H | n-$C_3H_7$ | $C_2H_5$ | |

| Verbin-dung Nr. | X | Z | $R^1$ | $R^2$ | Fp [°C]/$n_D$ (°C)/ $^1$H-NMR-Daten |
|---|---|---|---|---|---|
| 16 | $CH_2CH(OCH_3)CH_2CH_2CH_2$ | H | n-$C_3H_7$ | Allyl | |
| 17 | $CH_2CH_2CH(CH_3)CH_2CH_2$ | H | n-$C_3H_7$ | $C_2H_5$ | |
| 18 | $CH_2SCH_2SCH_2$ | H | $C_2H_5$ | $C_2H_5$ | 76-80 |
| 19 | $CH_2SCH_2SCH_2$ | H | $C_2H_5$ | Allyl | 1,5954 (22) |
| 20 | $CH_2SCH_2SCH_2$ | H | n-$C_3H_7$ | $C_2H_5$ | 80-82 |
| 21 | $CH_2SCH_2SCH_2$ | H | n-$C_3H_7$ | Allyl | 62-64 |
| 22 | $CH_2SCH_2SCH_2$ | H | $C_2H_5$ | 3-Chlor-allyl (trans) | |
| 23 | $CH_2SCH_2SCH_2$ | H | n-$C_3H_7$ | 3-Chlor-allyl (trans) | |
| 24 | $CH_2CH_2SCH_2CH_2$ | H | n-$C_3H_7$ | $C_2H_5$ | 1,5508 (23) |
| 25 | $CH_2CH_2SCH_2CH_2$ | H | n-$C_3H_7$ | 3-Chlor allyl (trans) | |
| 26 | $CH_2CH_2SCH_2CH_2$ | H | n-$C_3H_7$ | Allyl | 1,5558 (23) |
| 27 | $CH_2OCH_2CH_2CH_2$ | H | n-$C_3H_7$ | 3-Cl-Allyl (trans) | |
| 28 | $CH_2OCH_2CH_2CH_2$ | H | n-$C_3H_7$ | Methylallyl (trans) | |
| 29 | $CH_2OCH_2CH_2CH_2$ | H | $C_2H_5$ | $C_2H_5$ | |
| 30 | $CH_2OCH_2CH_2CH_2$ | H | $C_2H_5$ | Allyl | |
| 31 | $CH_2OCH_2CH_2CH_2$ | H | $C_2H_5$ | 3-Cl-Allyl (trans) | |
| 32 | $CH_2OCH_2CH_2CH_2$ | H | $C_2H_5$ | Methylallyl (trans) | |
| 33 | $CH_2CH_2OCH_2CH_2$ | H | n-$C_3H_7$ | 3-Cl-Allyl (trans) | |
| 34 | $CH_2CH_2OCH_2CH_2$ | H | n-$C_3H_7$ | Methlallyl (trans) | |
| 35 | $CH_2CH_2OCH_2CH_2$ | H | $C_2H_5$ | $C_2H_5$ | |
| 36 | $CH_2CH_2OCH_2CH_2$ | H | $C_2H_5$ | Allyl | |
| 37 | $CH_2CH_2OCH_2CH_2$ | H | $C_2H_5$ | 3-Cl-Allyl (trans) | |
| 38 | $CH_2CH_2OCH_2CH_2$ | H | $C_2H_5$ | Methylallyl (trans) | |
| 39 | $CH_2SCH_2CH_2CH_2$ | H | n-$C_3H_7$ | Methylallyl (trans) | |
| 40 | $CH_2SCH_2CH_2CH_2$ | H | $C_2H_5$ | $C_2H_5$ | |
| 41 | $CH_2SCH_2CH_2CH_2$ | H | $C_2H_5$ | Allyl | |
| 42 | $CH_2SCH_2CH_2CH_2$ | H | $C_2H_5$ | 3-Cl-Allyl (trans) | |
| 43 | $CH_2SCH_2CH_2CH_2$ | H | $C_2H_5$ | Methylallyl (trans) | |
| 44 | $SCH_2CH_2CH_2CH_2$ | H | n-$C_3H_7$ | $C_2H_5$ | |
| 45 | $SCH_2CH_2CH_2CH_2$ | H | n-$C_3H_7$ | Allyl | |
| 46 | $SCH_2CH_2CH_2CH_2$ | H | n-$C_3H_7$ | 3-Cl-Allyl (trans) | |
| 47 | $SCH_2CH_2CH_2CH_2$ | H | n-$C_3H_7$ | Methylallyl (trans) | |
| 48 | $SCH_2CH_2CH_2CH_2$ | H | $C_2H_5$ | $C_2H_5$ | |
| 49 | $SCH_2CH_2CH_2CH_2$ | H | $C_2H_5$ | Allyl | |
| 50 | $SCH_2CH_2CH_2CH_2$ | H | $C_2H_5$ | 3-Cl-Allyl (trans) | |
| 51 | $SCH_2CH_2CH_2CH_2$ | H | $C_2H_5$ | Methylallyl (trans) | |
| 52 | $CH_2CH_2SCH_2CH_2$ | H | n-$C_3H_7$ | 3-Cl-Allyl (trans) | |
| 53 | $CH_2CH_2SCH_2CH_2$ | H | n-$C_3H_7$ | Methylallyl (trans) | |
| 54 | $CH_2CH_2SCH_2CH_2$ | H | $C_2H_5$ | $C_2H_5$ | |
| 55 | $CH_2CH_2SCH_2CH_2$ | H | $C_2H_5$ | Allyl | |
| 56 | $CH_2CH_2SCH_2CH_2$ | H | $C_2H_5$ | 3-Cl-Allyl (trans) | |
| 57 | $CH_2CH_2SCH_2CH_2$ | H | $C_2H_6$ | Methylallyl (trans) | |
| 58 | $CH_2OCH_2OCH_2$ | H | n-$C_3H_7$ | $C_2H_5$ | |
| 59 | $CH_2OCH_2OCH_2$ | H | n-$C_3H_7$ | Allyl | |
| 60 | $CH_2OCH_2OCH_2$ | H | n-$C_3H_7$ | 3-Cl-Allyl (trans) | |
| 61 | $CH_2OCH_2OCH_2$ | H | n-$C_3H_7$ | Methylallyl (trans) | |
| 62 | $CH_2OCH_2OCH_2$ | H | $C_2H_5$ | $C_2H_5$ | |
| 63 | $CH_2OCH_2OCH_2$ | H | $C_2H_5$ | Allyl | |
| 64 | $CH_2OCH_2OCH_2$ | H | $C_2H_5$ | 3-Cl-Allyl (trans) | |
| 65 | $CH_2OCH_2OCH_2$ | H | $C_2H_5$ | Methylallyl (trans) | |

| Verbindung Nr. | X | Z | $R^1$ | $R^2$ | Fp [°C]/$n_D$ (°C)/ $^1$H-NMR-Daten |
|---|---|---|---|---|---|
| 66 | $CH_2SCH_2SCH_2$ | H | n-$C_3H_7$ | 3-Cl-Allyl (trans) | |
| 67 | $CH_2SCH_2SCH_2$ | H | n-$C_3H_7$ | Methylallyl (trans) | |
| 68 | $CH_2SCH_2SCH_2$ | H | $C_2H_5$ | 3-Cl-Allyl (trans) | |
| 69 | $CH_2SCH_2SCH_2$ | H | $C_2H_5$ | Methylallyl (trans) | |
| 70 | $CH_2CH(OCH_3)CH_2CH_2CH_2$ | H | n-$C_3H_7$ | $C_2H_5$ | |
| 71 | $CH_2CH(OCH_3)CH_2CH_2CH_2$ | H | n-$C_3H_7$ | Allyl | |
| 72 | $CH_2CH(OCH_3)CH_2CH_2CH_2$ | H | n-$C_3H_7$ | 3-Cl-Allyl (trans) | |
| 73 | $CH_2CH(OCH_3)CH_2CH_2CH_2$ | H | n-$C_3H_7$ | Methylallyl (trans) | |
| 74 | $CH_2CH(OCH_3)CH_2CH_2CH_2$ | H | $C_2H_5$ | $C_2H_5$ | |
| 75 | $CH_2CH(OCH_3)CH_2CH_2CH_2$ | H | $C_2H_5$ | Allyl | |
| 76 | $CH_2CH(OCH_3)CH_2CH_2CH_2$ | H | $C_2H_5$ | 3-Cl-Allyl (trans) | |
| 77 | $CH_2CH(OCH_3)CH_2CH_2CH_2$ | H | $C_2H_5$ | Methylallyl (trans) | |
| 78 | $CH_2CH_2CH(OCH_3)CH_2CH_2$ | H | n-$C_3H_7$ | $C_2H_5$ | |
| 79 | $CH_2CH_2CH(OCH_3)CH_2CH_2$ | H | n-$C_3H_7$ | Allyl | |
| 80 | $CH_2CH_2CH(OCH_3)CH_2CH_2$ | H | n-$C_3H_7$ | 3-Cl-Allyl (trans) | |
| 81 | $CH_2CH_2CH(OCH_3)CH_2CH_2$ | H | n-$C_3H_7$ | Methylallyl (trans) | |
| 82 | $CH_2CH_2CH(OCH_3)CH_2CH_2$ | H | $C_2H_5$ | $C_2H_5$ | |
| 83 | $CH_2CH_2CH(OCH_3)CH_2CH_2$ | H | $C_2H_5$ | Allyl | |
| 84 | $CH_2CH_2CH(OCH_3)CH_2CH_2$ | H | $C_2H_5$ | 3-Cl-Allyl (trans) | |
| 85 | $CH_2CH_2CH(OCH_3)CH_2CH_2$ | H | $C_2H_5$ | Methylallyl (trans) | |
| 86 | $CH(SCH_3)CH_2CH_2CH_2CH_2$ | H | n-$C_3H_7$ | $C_2H_5$ | |
| 87 | $CH(SCH_3)CH_2CH_2CH_2CH_2$ | H | n-$C_3H_7$ | Allyl | |
| 88 | $CH(SCH_3)CH_2CH_2CH_2CH_2$ | H | n-$C_3H_7$ | 3-Cl-Allyl (trans) | |
| 89 | $CH(SCH_3)CH_2CH_2CH_2CH_2$ | H | n-$C_3H_7$ | Methylallyl (trans) | |
| 90 | $CH(SCH_3)CH_2CH_2CH_2CH_2$ | H | $C_2H_5$ | $C_2H_5$ | |
| 91 | $CH(SCH_3)CH_2CH_2CH_2CH_2$ | H | $C_2H_5$ | Allyl | |
| 92 | $CH(SCH_3)CH_2CH_2CH_2CH_2$ | H | $C_2H_5$ | 3-Cl-Allyl (trans) | |
| 93 | $CH(SCH_3)CH_2CH_2CH_2CH_2$ | H | $C_2H_5$ | Methylallyl (trans) | |
| 94 | $CH_2CH(SCH_3)CH_2CH_2CH_2$ | H | n-$C_3H_7$ | $C_2H_5$ | |
| 95 | $CH_2CH(SCH_3)CH_2CH_2CH_2$ | H | n-$C_3H_7$ | Allyl | |
| 96 | $CH_2CH(SCH_3)CH_2CH_2CH_2$ | H | n-$C_3H_7$ | 3-Cl-Allyl (trans) | |
| 97 | $CH_2CH(SCH_3)CH_2CH_2CH_2$ | H | n-$C_3H_7$ | Methylallyl (trans) | |
| 98 | $CH_2CH(SCH_3)CH_2CH_2CH_2$ | H | $C_2H_5$ | $C_2H_5$ | |
| 99 | $CH_2CH(SCH_3)CH_2CH_2CH_2$ | H | $C_2H_5$ | Allyl | |
| 100 | $CH_2CH(SCH_3)CH_2CH_2CH_2$ | H | $C_2H_5$ | 3-Cl-Allyl (trans) | |
| 101 | $CH_2CH(SCH_3)CH_2CH_2CH_2$ | H | $C_2H_5$ | Methylallyl (trans) | |
| 102 | $CH_2CH(SCH_2CH_3)CH_2CH_2CH_2$ | H | n-$C_3H_7$ | 3-Cl-Allyl (trans) | |
| 103 | $CH_2CH(SCH_2CH_3)CH_2CH_2CH_2$ | H | n-$C_3H_7$ | Methylallyl (trans) | |
| 104 | $CH_2CH(SCH_2CH_3)CH_2CH_2CH_2$ | H | $C_2H_5$ | $C_2H_5$ | |
| 105 | $CH_2CH(SCH_2CH_3)CH_2CH_2CH_2$ | H | $C_2H_5$ | Allyl | |
| 106 | $CH_2CH(SCH_2CH_3)CH_2CH_2CH_2$ | H | $C_2H_5$ | 3-Cl-Allyl (trans) | |
| 107 | $CH_2CH(SCH_2CH_3)CH_2CH_2CH_2$ | H | $C_2H_5$ | Methylallyl (trans) | |
| 108 | $CH_2CH(SOCH_3)CH_2CH_2CH_2$ | H | n-$C_3H_7$ | $C_2H_5$ | |
| 109 | $CH_2CH(SOCH_3)CH_2CH_2CH_2$ | H | n-$C_3H_7$ | Allyl | |
| 110 | $CH_2CH(SOCH_3)CH_2CH_2CH_2$ | H | n-$C_3H_7$ | 3-Cl-Allyl (trans) | |
| 111 | $CH_2CH(SOCH_3)CH_2CH_2CH_2$ | H | n-$C_3H_7$ | Methylallyl (trans) | |
| 112 | $CH_2CH(SOCH_3)CH_2CH_2CH_2$ | H | $C_2H_5$ | $C_2H_5$ | |
| 113 | $CH_2CH(SOCH_3)CH_2CH_2CH_2$ | H | $C_2H_5$ | Allyl | |
| 114 | $CH_2CH(SOCH_3)CH_2CH_2CH_2$ | H | $C_2H_5$ | 3-Cl-Allyl (trans) | |
| 115 | $CH_2CH(SOCH_3)CH_2CH_2CH_2$ | H | $C_2H_5$ | Methylallyl (trans) | |

| Verbindung Nr. | X | Z | $R^1$ | $R^2$ | Fp [°C]/$n_D$ (°C)/ $^1$H-NMR-Daten |
|---|---|---|---|---|---|
| 116 | $CH_2CH(SO_2CH_3)CH_2CH_2CH_2$ | H | n-$C_3H_7$ | $C_2H_5$ | |
| 117 | $CH_2CH(SO_2CH_3)CH_2CH_2CH_2$ | H | n-$C_3H_7$ | Allyl | |
| 118 | $CH_2CH(SO_2CH_3)CH_2CH_2CH_2$ | H | n-$C_3H_7$ | 3-Cl-Allyl (trans) | |
| 119 | $CH_2CH(SO_2CH_3)CH_2CH_2CH_2$ | H | n-$C_3H_7$ | Methylallyl (trans) | |
| 120 | $CH_2CH(SO_2CH_3)CH_2CH_2CH_2$ | H | $C_2H_5$ | $C_2H_5$ | |
| 121 | $CH_2CH(SO_2CH_3)CH_2CH_2CH_2$ | H | $C_2H_5$ | Allyl | |
| 122 | $CH_2CH(SO_2CH_3)CH_2CH_2CH_2$ | H | $C_2H_5$ | 3-Cl-Allyl (trans) | |
| 123 | $CH_2CH(SO_2CH_3)CH_2CH_2CH_2$ | H | $C_2H_5$ | Methylallyl (trans) | |
| 124 | $CH_2CH_2N(COCH_3)CH_2CH_2$ | H | n-$C_3H_7$ | $C_2H_5$ | |
| 125 | $CH_2CH_2N(COCH_3)CH_2CH_2$ | H | n-$C_3H_7$ | Allyl | |
| 126 | $CH_2CH_2N(COCH_3)CH_2CH_2$ | H | n-$C_3H_7$ | 3-Cl-Allyl (trans) | |
| 127 | $CH_2CH_2N(COCH_3)CH_2CH_2$ | H | n-$C_3H_7$ | Methylallyl (trans) | |
| 128 | $CH_2CH_2N(COCH_3)CH_2CH_2$ | H | $C_2H_5$ | $C_2H_5$ | |
| 129 | $CH_2CH_2N(COCH_3)CH_2CH_2$ | H | $C_2H_5$ | Allyl | |
| 130 | $CH_2CH_2N(COCH_3)CH_2CH_2$ | H | $C_2H_5$ | 3-Cl-Allyl (trans) | |
| 131 | $CH_2CH_2N(COCH_3)CH_2CH_2$ | H | $C_2H_5$ | Methylallyl (trans) | |
| 132 | $CH_2CH_2N(COC_6H_5)CH_2CH_2$ | H | n-$C_3H_7$ | $C_2H_5$ | 83-85 |
| 133 | $CH_2CH_2N(COC_6H_5)CH_2CH_2$ | H | n-$C_3H_7$ | Allyl | |
| 134 | $CH_2CH_2N(COC_6H_5)CH_2CH_2$ | H | n-$C_3H_7$ | 3-Cl-Allyl (trans) | |
| 135 | $CH_2CH_2N(COC_6H_5)CH_2CH_2$ | H | n-$C_3H_7$ | Methylallyl (trans) | |
| 136 | $CH_2CH_2N(COC_6H_5)CH_2CH_2$ | H | $C_2H_5$ | $C_2H_5$ | |
| 137 | $CH_2CH_2N(COC_6H_5)CH_2CH_2$ | H | $C_2H_5$ | Allyl | |
| 138 | $CH_2CH_2N(COC_6H_5)CH_2CH_2$ | H | $C_2H_5$ | 3-Cl-Allyl (trans) | |
| 139 | $CH_2CH_2N(COC_6H_5)CH_2CH_2$ | H | $C_2H_5$ | Methylallyl (trans) | |
| 140 | $CH_2CH_2N(COC_6H_5)CH_2CH_2$ | $CO_2CH_3$ | n-$C_3H_5$ | $C_2H_5$ | 55-57°C |
| 141 | $CH_2CH_2N(COCH_3)CH_2CH_2$ | $CO_2CH_3$ | n-$C_3H_7$ | $C_2H_5$ | 0,98 (t,3H); 1,35 (t,3H); 1,47-1,74 (m,6H); 2,09 (s,3H); 2,87-3,0 (m,2H); 3,32-3,6 (m,2H); 3,72 (s,3H); 4,1 (q,2H) |
| 142 | $CH_2CH_2CH(SCH_3)CH_2CH_2$ | H | n-$C_3H_7$ | $C_2H_5$ | |
| 143 | $CH_2CH_2CH(SCH_3)CH_2CH_2$ | H | n-$C_3H_7$ | Allyl | |
| 144 | $CH_2CH_2CH(SCH_3)CH_2CH_2$ | H | n-$C_3H_7$ | 3-Cl-Allyl (trans) | |
| 145 | $CH_2CH_2CH(SCH_3)CH_2CH_2$ | H | n-$C_3H_7$ | Methylallyl (trans) | |
| 146 | $CH_2CH_2CH(SCH_3)CH_2CH_2$ | H | $C_2H_5$ | $C_2H_5$ | |
| 147 | $CH_2CH_2CH(SCH_3)CH_2CH_2$ | H | $C_2H_5$ | Allyl | |
| 148 | $CH_2CH_2CH(SCH_3)CH_2CH_2$ | H | $C_2H_5$ | 3-Cl-Allyl (trans) | |
| 149 | $CH_2CH_2CH(SCH_3)CH_2CH_2$ | H | $C_2H_5$ | Methylallyl (trans) | |

Die Cyclohexenone der Formel I können beispielsweise in Form von direkt versprühbaren Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten oder Öldispersionen kommen Mineralölfraktionen von mittleren Naphthalinen oder Methanol, Ethanol, Propanol, Butanol, Chloroform, Tetrachlorkohlenstoff, Cyclohexanol, Cyclohexanon, Chlorobenzol, Isophoron, stark polare Lösungsmittel, wie Dimethylformamid, Dimethylsulfoxid, N-Methylpyrrolidon, Wasser, in Betracht.

Wässrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäure mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiel für Formulierungen sind:

I. Man vermischt 90 Gewichtsteile der Verbindung Nr. 2 mit 10 Gewichtsteilen N-Methyl-λ-pyrrolidon und erhält eine Lösung, die zur Anwendung in Form kleinster Tropfen geeignet ist.

II. 20 Gewichtsteile der Verbindung Nr. 8 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 6 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässrige Dispersion, die 0,02 Gewichtsprozent des Wirkstoffs enthält.

III. 20 Gewichtsteile der Verbindung Nr. 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

IV. 20 Gewichtsteile des Wirkstoffs Nr. 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

V. 20 Gewichtsteile des Wirkstoffs Nr. 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile des Wirkstoffs Nr. 9 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 9 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf Weise eine Aufbereitung des Wirkstoffs mit guter Haftfähigkeit.

VIII. 20 Teile des Wirkstoffs Nr. 6 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (postdirect, lay-by).

Die Aufwandmengen an Wirkstoff betragen je nach Jahreszeit, Zielpflanzen und Wachstumsstadium 0,025 bis 3 kg/ha, vorzugsweise 0,1 bis 0,5 kg/ha.

Die Wirkung der Cyclohexenone der Formel I auf das Pflanzenwachstum lässt sich durch Gewächshausversuche zeigen:

Als Kulturgefässe dienen Plastikblumentöpfe mit 300 $cm^3$ Inhalt und lehmigem Sand mit etwa 1,5% Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefässe leicht beregnet, um Keimung und Wachstum in Gang zu bringen. Danach deckt man die Gefässe mit durchsichtigen Plastikhauben ab, bis die Pflanzen angewachsen sind. Diese Abdeckung bewirkt ein gleichmässiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelte sie danach. Die Sojapflanzen werden in einem mit Torfmull (peat) angereicherten Substrat angezogen. Zur Nachauflaufbehandlung werden entweder direkt gesäte und in den gleichen Gefässen aufgewachsene Pflanzen ausgewählt, oder sie werden erst als Keimpflanzen getrennt angezogen und einige Tage vor der Behandlung in die Versuchsgefässe verpflanzt. Die Aufwandmengen für die Nachauflaufbehandlung betragen 0,125 bzw. 0,25 kg Wirkstoff/ha. Eine Abdeckung unterbleibt bei der Nachauflaufbehandlung.

Die Versuchsgefässe werden im Gewächshaus aufgestellt, wobei für wärmeliebende Arten wärmere Bereiche (20 bis 35°C) und für solche gemässigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Avena fatua (Flughafer), Avena sativa (Hafer), Digitaria sanguinalis (Blutfingerhirse), Echinochloa crus-galli (Hühnerhirse), Glycine max (Sojabohnen), Hordeum vulgare (Gerste), Lolium multiflorum (Ital. Raygras), Setaria italica (Kolbenhirse), Sinapis alba (Weisser Senf), Oryza sativa (Reis).

Vergleichsmittel sind Herbizide, die die bekannten Wirkstoffe 3-(1-Ethoxyimino-n-butyl)-spiro[5.5]undecan-2,4-dion (A; JP-OS 82/183 746) oder 8-(1-Ethoxyimino-n-butyl)-2-thia-spiro[4.5]decan-7,9-dion (B; JP-OS 83/144 348).

Vorauflaufanwendung:

Bei der Vorauflaufanwendung erweisen sich beispielsweise die Verbindungen Nr. 1, 2, 6, 8 und 9 als herbizid wirksam gegen Pflanzen aus der Familie der Gräser, während weisser Senf (Sinapis alba) als Vertreter dikotyler Pflanzen völlig ungeschädigt bleibt.

Nachauflaufanwendung:

Bei Nachauflaufanwendung von 0,125 kg Wirkstoff/ha bekämpfen beispielsweise die Verbindungen Nr. 1 und 2 grasartige Pflanzen besser als das Vergleichsmittel A, wobei Sojabohnen als dikotyle Kulturpflanzen nicht geschädigt werden. Bei der Bekämpfung unerwünschter Grasarten in Reis, einer Kultur aus der Familie der Gräser, sind beispielsweise die Verbindungen Nr. 6 und 8 verträglicher als das Vergleichsmittel 8.

Mit den Verbindungen Nr. 24 und 26 lassen sich bedeutende Problemungräser in Sojakulturen besser bekämpfen als mit dem bekannten Wirkstoff A; die breitblättrige Nutzpflanze erleidet dabei keinen Schaden. Die Verbindungen Nr. 20 und 21 beispielsweise eignen sich zum Einsatz gegen Ausfallkulturen aus der Familie der Gramineen, wie Sorghum bicolor und Zea mays, sowie gegen wichtige Ungräser in Sojakulturen. Das Vergleichsmittel A zeigt dabei eine deutlich niedrigere herbizide Aktivität.

In Anbetracht des erfassbaren Wirkungsspektrums zur Unkrautbekämpfung, der Verträglichkeit für Kulturpflanzen oder der unerwünschten Beeinflussung des Wachstums derselben sowie angesichts der Vielfalt der Applikationsmethoden können die erfindungsgemässen Verbindungen in einer grossen Zahl von Kulturpflanzen eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuss |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weisse Rübe |
| Brassica rapa var. silvestris | Rübsen |
| Camellia sinensis | Teestrauch |
| Carthamus tinctorius | Saflor - Färberdistel |
| Carya illinoinensis | Pekannussbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |

| Botanischer Name | Deutscher Name |
|---|---|
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süsskartoffeln |
| Juglans regia | Walnussbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europea | Ölbaum |
| Oryza sativa | Reis |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |
| Picea abies | Rotfichte |
| Abies alba | Weisstanne |
| Pinus spp. | Kiefer |
| Pisum sativum | Gartenerbse |
| Prunus avium | Süsskirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preisselbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenone der Formel I sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolin-carbonsäuren, Cyclohexenone anderer Struktur und andere Wirkstoffe in Betracht.

Ausserdem kann es von Nutzen sein, die Cyclohexenone der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nicht-phytotoxische Öle und Ölkonzentrate zugesetzt werden.

**Patentansprüche**

1. Cyclohexenone der Formel

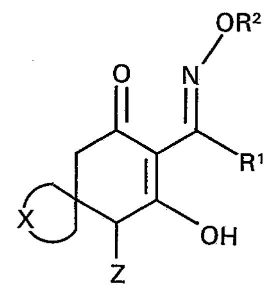

(I)

in der

$R^1$ $C_1$-$C_4$-Alkyl,

$R^2$ $C_1$-$C_4$-Alkyl, $C_3$-$C_5$-Alkinyl oder gegebenenfalls halogensubstituiertes $C_3$-$C_5$-Alkenyl,

Z Wasserstoff oder $C_2$-$C_5$-Alkoxycarbonyl und

X eine gegebenenfalls durch $C_1$-$C_4$-Alkyl, $C_1$-$C_4$-Alkoxy oder $C_1$-$C_4$-Alkylthio substituierte Pentamethylenkette, in der eine oder zwei Methylengruppen durch O, S, SO, $SO_2$ oder $NR^3$, wobei $R^3$ für $C_1$-$C_4$-Alkyl, $C_2$-$C_7$-Acyl oder Benzyl steht, ersetzt sein können, mit der Massgabe, dass ein Substituent aus der Gruppe bestehend aus Alkyl, Alkoxy und Alkylthio vorhanden ist, wenn X Pentamethylen ist, bedeuten.

2. Cyclohexenone der Formel I gemäss Anspruch 1, dadurch gekennzeichnet, dass Z Wasserstoff bedeutet.

3. Cyclohexenone der Formel I gemäss Anspruch 1, dadurch gekennzeichnet, dass X eine Oxapentamethylen- oder eine Thiapentamethylenkette bedeutet.

4. 9-(1-Ethoxyimino-n-butyl)-3-oxa-spiro[5.5]-undecan-8,10-dion.

5. Verfahren zur Herstellung eines Cyclohexenons der Formel I gemäss Anspruch 1, dadurch gekennzeichnet, dass man eine Verbindung der Formel

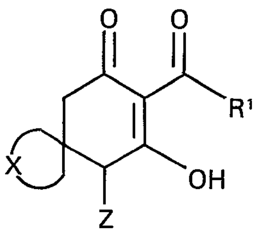

(II)

in der $R^1$, Z und X die in Anspruch 1 genannten Bedeutungen haben,

a) mit einer Ammoniumverbindung der Formel $R^2ONH_3Y$, in der $R^2$ die in Anspruch 1 genannten Bedeutungen hat und Y ein beliebiges Anion bedeutet, in einem inerten Lösungsmittel bei einer Temperatur zwischen 0 und 80°C gegebenenfalls unter Zugabe einer Hilfsbase umsetzt, oder

b) mit einem gegebenenfalls in wässriger Lösung vorliegenden Hydroxylamin der Formel $R^2ONH_2$, wobei $R^2$ die in Anspruch 1 genannten Bedeutungen hat, bei Temperaturen zwischen 0 und 80°C gegebenenfalls unter Zugabe eines Lösungsmittels umsetzt, oder

c) mit unsubstituiertem Hydroxylammoniumsalz $NH_2OH \cdot HY$, wobei Y die obige Bedeutung hat, bei 0 bis 80°C unter Zugabe von Lösungsmittel und Hilfsbase umsetzt und das so erhaltene Oxim mit einem Alkylierungsmittel $R^2$-Y′, wobei $R^2$ die in Anspruch 1 genannten Bedeutungen hat und Y′ eine Abgangsgruppe bedeutet, alkyliert.

6. Herbizid, enthaltend ein Cycloohexenon der Formel I gemäss Anspruch 1.

7. Herbizid, enthaltend inerte Zusatzstoffe und ein Cyclohexenon der Formel I gemäss Anspruch 1.

8. Herbizid nach Anspruch 7, dadurch gekennzeichnet, dass es 0,1 bis 95 Gew.-% Cyclohexenon der Formel I enthält.

9. Verfahren zur Bekämpfung unerwünschten Pflanzenwuchses, dadurch gekennzeichnet, dass man die unerwünschten Pflanzen oder von unerwünschten Pflanzenwachstum freizuhaltene Fläche mit einer herbizid wirksamen Menge eines Cyclohexenons der Formel I gemäss Anspruch 1 behandelt.

10. Verfahren nach Anspruch 9, dadurch gekennzeichnet, dass die Aufwandmenge an Cyclohexenon der Formel I 0,025 bis 3 kg/ha beträgt.

**Claims**

1. A cyclohexenone of the formula

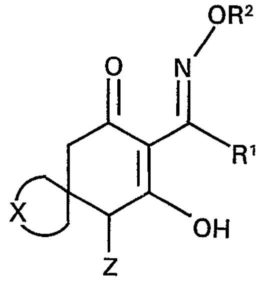

(I)

where

$R^1$ is $C_1$-$C_4$-alkyl,

$R^2$ is $C_1$-$C_4$-alkyl, $C_3$-$C_5$-alkynyl or unsubstituted or halogen-substituited $C_3$-$C_5$-alkenyl,

Z is hydrogen or $C_2$-$C_5$-alkoxycarbonyl and

X is a pentamethylene chain which is unsubstituted or substituted by $C_1$-$C_4$-alkyl, $C_1$-$C_4$-alkoxy or $C_1$-$C_4$-alkylthio and in which one or two methylene groups may be replaced by O, S, SO, $SO_2$ or $NR^3$, where $R^3$ is $C_1$-$C_4$-alkyl, $C_2$-$C_7$-acyl or benzyl, with the proviso that one substituent from the group consisting of alkyl, alkoxy or alkylthio is present when X is pentamethylene.

2. A cyclohexenone of the formula I as claimed in claim 1, where Z is hydrogen.

3. A cyclohexenone of the formula I as claimed in claim 1, where X is an oxapentamethylene or thiapentamethylene chain.

4. 9-(1-Ethoxyimino-n-butyl)-3-oxaspiro[5,5]-undecane-8,10-dione.

5. Process for the preparation of a cyclohexenone of the formula I as claimed in claim 1, wherein a compound of the formula

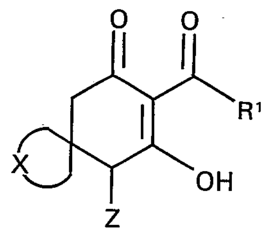

(II)

where $R^1$, Z and X have the meanings stated in claim 1,

a) is reacted with an ammonium compound of the formula $R^2ONH_3Y$, where $R^2$ has the meanings stated in claim 1 and Y is any anion, in an inert solvent at from 0 to 80°C, with or without the addition of an auxiliary base, or

b) is reacted with a hydroxylamine of the formula $R^2ONH_2$, where $R^2$ has the meanings stated in claim 1, at from 0 to 80°C with or without the addition of a solvent, if appropriate the said hydroxylamine being present in aqueous solution, or

c) is reacted with an unsubstituted hydroxylammonium salt $NH_2OH \cdot HY$, where Y has the above meaning, at from 0 to 80°C, with the addition of a solvent and of an auxiliary base, and the oxime thus obtained is alkylated with an alkylating agent $R^2$-Y′, where $R^2$ has the meanings stated in claim 1 and Y′ is a leaving group.

6. A herbicide containing a cyclohexenone of the formula I as claimed in claim 1.

7. A herbicide containing inert additives and a cyclohexenone of the formula I as claimed in claim 1.

8. A herbicide as claimed in claim 7, containing from 0.1 to 95% by weight of a cyclohexenone of the formula I.

9. A process for combating unwanted plant growth, wherein the unwanted plants or the area to be kept free from unwanted plant growth are treated with a herbicidally effective amount of a cyclohexenone of the formula I as claimed in claim 1.

10. A process as claimed in claim 9, wherein the cyclohexenone of the formula is applied at a rate of from 0.025 to 3 kg/ha.

**Revendications**

1. Cyclohexénone de la formule

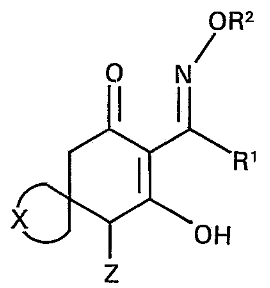

(I)

dans laquelle ,.
$R^1$ désigne un radical alkyle en $C_1$ à $C_4$,
$R^2$ désigne un radical alkyle en $C_1$ à $C_4$, alcynyle en $C_3$ à $C_5$ ou alcényle en $C_3$ à $C_5$ éventuellement halosubstitué,
Z représente un atome d'hydrogène ou un groupe alcoxycarbonyle en $C_2$ à $C_5$ et
X représente une chaîne pentaméthylène éventuellement alkyl(en $C_1$ à $C_4$)-, alcoxy(en $C_1$ à $C_4$)- ou alkyl(en $C_1$ à $C_4$)-thio-substituée, dans laquelle un ou deux chaînons méthylène peuvent être remplacés par O, S, SO, $SO_2$ ou $NR^3$, où $R^3$ désigne un radical alkyle en $C_1$ à $C_4$ ou un groupe acyle en $C_2$ à $C_7$ ou benzyle, au moins un substituant alkyle, alcoxy ou alkylthio devant être présent, si X = pentaméthylène.

2. Cyclohexénones de la formule I selon la revendication 1, caractérisées en ce que Z = H.

3. Cyclohexénones de la formule I selon la revendication 1, caractérisées en ce que X désigne une chaîne oxapentaméthylène ou thiapentaméthylène.

4. La 9-(1-éthoxyimino-n-butyl)-3-oxa-spiro 5,5-undécane-8,10-dione.

5. Procédé de préparation d'une cyclohexénone de la formule I selon la revendication 1, caractérisé en ce que l'on fait réagir un composé de la formule

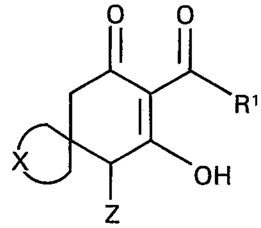

(II)

dans laquelle $R^1$, Z et X possèdent les significations définies dans la revendication 1,

a) dans un solvant inerte à une température comprise entre 0 et 80°C avec un dérivé d'ammonium de la formule $R^2ONH_3Y$, dans laquelle $R^2$ possède les significations définies dans la revendication 1 et Y représente un anion quelconque, éventuellement en présence d'une base auxiliaire, ou

b) à des températures comprises entre 0 et 80°C, éventuellement en présence d'un solvant, avec une hydroxylamine de la formule $R^2ONH_2$, dans laquelle $R^2$ possède les significations définies dans la revendication 1, éventuellement mise en œuvre en solution aqueuse, ou

c) entre 0 et 80°C, en présence d'un solvant et d'une base auxiliaire, avec un sel d'hydroxylammonium non substitué de la formulae $NH_2OH \cdot HY$, où possède la signification définie ci-dessus, l'oxime formée étant ensuite alkylée à l'aide d'un agent d'alkylation de la formule $R^2$ - Y', dans laquelle $R^2$ possède les significations définies dans la revendication 1 et y' représente un groupe à cliver.

6. Composition herbicide, contenant une cyclohexénone de la formule I selon la revendication 1.

7. Composition herbicide, contenant une cyclohéxenone de la formule I selon la revendication 1, ainsi que des additifs inertes.

8. Composition herbicide suivant la revendication 7, caractérisée par une teneur en cyclohexénone de la formule I de 0,1 à 95% en poids.

9. Procédé de lutte contre le développement de plantes indésiderables, caractérisé en ce que l'on traite les plantes indésirables ou les surfaces, sur lesquelles le développement de plantes indésirables doit être inhibé, avec une quantité efficace du point de vue herbicide d'une cyclohexénone de la formule I selon la revendication 1.

10. Procédé suivant la revendication 9, caractérisé en ce que la quantité d'application d'une cyclohexénone de la formule I se situe entre 0,025 et 3 kg par ha.